# EUROPEAN PATENT SPECIFICATION

(11) **EP 2 588 067 B1**
(45) Date of publication and mention of the grant of the patent: **26.10.2016**
(21) Application number: 11711963.6
(22) Date of filing: 22.03.2011
(51) Int. Cl.: A61K 8/19, A61K 8/21, A61K 8/27, A61K 8/44, A61K 8/66, A61K 8/67, A61K 8/97, A61Q 11/00, A61K 8/34

(54) **COMPOSITION FOR DENTAL HEALTH**
ZUSAMMENSETZUNG FÜR MUNDGESUNDHEIT
COMPOSITION POUR SOIN BUCCAL

(30) Priority: 01.07.2010 GB 201011074
(43) Date of publication of application: 08.05.2013
(73) Proprietor: Taylor, Robert Peter, Little Venice London W9 1AH (GB); LALVANI, Tej, London W1G 9YA (GB); SHELATKAR, Rohit, Maida Vale London W9 3SP (GB)
(72) Inventor: Taylor, Robert Peter, Little Venice London W9 1AH (GB); LALVANI, Tej, London W1G 9YA (GB); SHELATKAR, Rohit, Maida Vale London W9 3SP (GB)
(86) International application number: PCT/GB2011/000399
(87) International publication number: WO 2012/001337

(56) References cited:
- WO-A2-02/02128
- WO-A2-2006/109344
- WO-A2-2009/043588
- US-A1- 2005 079 140
- US-A1- 2007 053 849
- US-A1- 2007 122 362
- US-A1- 2007 231 277
- US-B1- 6 372 198
- DATABASE WPI Week 200164 Thomson Scientific, London, GB; AN 2001-569531 XP002661799, & KR 2001 0028609 A (OH S G) 6 April 2001 (2001-04-06)
- DATABASE WPI Week 200961 Thomson Scientific, London, GB; AN 2009-M94126 XP002661800, & CN 101 502 482 A (YUXI JIANKUN BIOLOGICAL PHARM CO LTD) 12 August 2009 (2009-08-12)
- MATIVANDLELA S P N ET AL: "ANTIMICROBIAL ACTIVITY OF PELARGONIUM SIDOIDES", SOUTH AFRICAN JOURNAL OF BOTANY - SUID-AFRIKAANS TYDSKRIFT VIRPLANTKUNDE, FOUNDATION FOR EDUCATION, SCIENCE AND TECHNOLOGY, PRETORIA, SA, vol. 70, no. 2, 1 May 2004 (2004-05-01), page 344, XP009074186, ISSN: 0254-6299
- DATABASE GNPD MINTEL 10231717, 01 September 2005 'Antibacterial mouthwash'
- DATABASE GNPD [Online] MINTEL 03 March 2008 'Toothpaste' Database accession no. 874992
- DATABASE GNPD [Online] MINTEL 01 September 2008 'Nano-whitening toothpaste' Database accession no. 976003
- DATABASE GNPD [Online] MINTEL 03 December 2007 'All Natural Ester CoQ10 Tooth Gel' Database accession no. 826739

## Description

### TECHNICAL FIELD

This invention concerns a composition, combined with standard constituents of toothpastes and mouthwashes for use in dental and oral hygiene, infections and inflammations.

### BACKGROUND ART

### Toothpaste

Toothpaste is a paste or gel dentifrice used with a toothbrush as an accessory to clean and maintain the aesthetics and health of teeth. It aids in removing dental plaque and food from the teeth, helps to eliminate or mask halitosis, and delivers active ingredients such as fluoride or xylitol in order to prevent tooth and gum disease (gingivitis). Most of the cleaning is done by the mechanical use of the toothbrush.

Fluoride in various forms is the most popular active ingredient in toothpaste. It is included in toothpastes in order to prevent cavities. It occurs naturally in small amounts in plants and animals. Sodium fluoride is the most common form of flouride used in toothpastes. Some brands of toothpaste use sodium monofluorophosphate. Other ingredients are less commonly used, including Hydroxyapatite nanocrystals and calcium phosphate for remineralisation of teeth, and potassium nitrate to reduce sensitivity. Triclosan, which is an antibacterial agent, is an active ingredient in some toothpastes, that is used to prevent gingivitis.

In addition to fluoride, the other fundamental ingredient in most toothpastes is an abrasive. Abrasives in toothpaste reduce the time needed to remove plaque from the teeth by half. Abrasives, like the dental polishing agents used in dental surgeries, also cause a small amount of enamel erosion which is termed "polishing" action. Some brands contain powdered white mica, which acts as a mild abrasive, and adds a cosmetically pleasing glittery shimmer to the paste. Removal of plaque and calculus prevents caries and periodontal disease. The polishing of teeth removes stains from tooth surfaces, but has not been shown to improve dental health beyond the effects of the removal of plaque and calculus.

Many, though not all, toothpastes contain sodium lauryl sulfate. It is largely a foaming agent although it also acts as a powerful antimicrobial. However, sodium lauryl sulfate may cause a greater frequency of mouth ulcers in some people as it can dry out the protective layer of oral tissues causing the underlying tissues to become damaged.

Toothpaste comes in a variety of colours and flavours. The more usual flavorings are some variation on mint, spearmint, peppermint, and regular mint. More exotic flavours include : anise, apricot, cinnamon, fennel, lavender, neem, ginger, vanilla, lemon, orange, pine. Unflavoured toothpaste does exist, however, most are flavoured and sweetened. Because sugar promotes growth of bacteria that cause tooth decay, artificial sweeteners such as sorbitol or saccharin are generally used instead.

### Mouthwash

Mouthwash (or mouth rinse) is a product used to enhance oral hygiene. Antiseptic and anti-plaque mouth rinse claims to kill the dental plaque causing dental caries, gingivitis, and bad breath. Anti-cavity mouth rinse uses fluoride to protect against tooth decay. However, the use of mouthwash does not eliminate the need for both brushing and flossing.

Active ingredients in commercial brands of mouthwash can include thymol, eucalyptol, methylparaben, hydrogen peroxide, domiphen bromide, fluoride, enzymes and calcium. Ingredients also include water, sweeteners such as sorbitol, sucralose, sodium saccharine, and xylitol, which doubles as a bacterial inhibitor. Sometimes a significant amount of ethanol is added. Commercial mouthwashes usually contain a preservative such as sodium benzoate and methylparaben to preserve freshness once the container has been opened. Many newer brands are alcohol free and contain odour elimination agents such as oxidizers to keep future bad breath from developing.

### Oral hygiene products

The following are the formulations of commonly used oral and dental hygiene products :
Sensodyne Multiaction with iso-active® technology (general cleansing) - Active ingredients : Potassium Nitrate 5% (for antihypersensitivity), Sodium fluoride 0.15% w/v fluoride ion (for anticavity); Inactive ingredients : Water, sorbitol, glycerin, hydrated silica, PEG-8, sodium methyl cocoyl taurate, isopentane, flavor, cocamidopropyl betaine, xanthan gum, carrageenan, sodium saccharin, sucralose, sodium hydroxide, FD&C blue #1.
Sensodyne Multiaction with iso-active® technology (general cleansing & whitening) - Active ingredients : Potassium Nitrate 5% (for anti -hypersensitivity), Sodium fluoride 0.15% w/v fluoride ion (for anticavity); Inactive ingredients : Water, glycerin, sorbitol, hydrated silica, pentasodium triphosphate, PEG-8, isopentane, flavor, sodium methyl cocoyl taurate, cocamidopropyl betaine, xanthan gum carrageenan, sodium hydroxide, sodium saccharin, sucralose, FD & C Blue, # 1.
Sensodyne Pronamel - Active ingredients : Potassium nitrate 5% w/w, Sodium Fluoride (1450 ppm fluoride); Inactive ingredients : Water, glycerin, sorbitol, hydrated silica, PEG-6, cocamidopropyl betaine, xanthan gum carrageenan, sodium hydroxide, sodium saccharin, aroma, titanium dioxide, limonene, anise alcohol.
Sensodyne TOTAL CARE (gentle whitening) - Active ingredients : Potassium nitrate, Sodium Fluoride (1400 ppm fluoride); Inactive Ingredients : Water, glycerin, sorbitol, hydrated silica, pentasodium triphosphate, PEG-6, cocamidopropyl betaine, xanthan gum carrageenan, sodium hydroxide, sodium saccharin, aroma, titanium dioxide, sodium methyl cocoyl taurate.

Pearl Drops-replenishing white - Ingredients : Sodium Bicarbonate (Baking Soda), Glycerin, PEG-8, Hydrated Silica, Silica, PEG/PPG-116/66 Copolymer, Calcium Sulphate, Sodium Lauryl Sulfate, Aroma, Dipotassium Phosphate, Sodium Carbonate, Sodium Saccharin, Cellulose Gum, Sodium Fluoride, Limonene, CI 77891.

Pearl Drops-party sparkle - Ingredients : Aqua, Sorbitol, Hydrated Silica, Glycerin, Sodium Hexametaphosphate, Sodium Lauryl Sulfate, PEG-12, Aroma, Sodium Monofluorophosphate, Cellulose Gum, Sodium Saccharin, Mica, Cinnamal, Benzyl Alcohol, CI 42053, CI 77891. Contains Sodium Monofluorophosphate (1000 ppm F).

Pearl Drops-Hollywood smile - Ingredients : Sorbitol, Aqua, Hydrated Silica, Glycerin, Alumina, Sodium Dodecylbenzenesulfonate, PEG-12, Aroma, Pentasodium Triphosphate, Pentapotassium Triphosphate, Zinc Peroxide, Cellulose Gum, Sodium Fluoride, Sodium Saccharin, Pearl Powder, Limonene, CI 74160, CI 77891. Contains Sodium Fluoride (1300 ppm F).

Swiss Dent- Stainless extensive bleaching- RDA 40 - Ingredients : Nanoxyd, Calcium peroxide, papain, bromelain, Fluoride, Coenzyme Q10, vitamin E, baking soda.

Swiss Dent- Nanowhitening gentle bleaching- RDA 25 - Ingredients : Nanoxyd, Calcium peroxide, papain, bromelain, Fluoride, Coenzyme Q10, vitamin E, baking soda.

Swissdent Purefreshness Breath Control - Contains sodium fluoride. Ingredients : Nanoxyd, Calcium peroxide, papain, bromelain, Fluoride, Coenzyme Q10, vitamin E, baking soda.

Retardex® - Active Ingredients : CloSYS II brand of Stabilised Chlorine Dioxide, Aqua, Hydrated Silica, Sorbitol, Glycerin, Cellulose Gum, Trisodium Phosphate, Sodium Saccharin, Mentha Piperita, Mentha Spicata, Menthol, Sodium Bicarbonate, Sodium Chlorite, Limonene, CI 77891/Titanium Dioxide. Arm and Hammer- enamel care (For natural whitening) - Ingredients : Sodium Bicarbonate (Baking Soda), Glycerin, PEG-8, Hydrated Silica, Silica, PEG/PPG-116/66 Copolymer, Calcium Sulfate, Sodium Lauryl Sulfate, Aroma, Dipotassium Phosphate, Sodium Saccharin, Cellulose Gum, Sodium Fluoride, Limonene, C142053, C1 77891.

Arm and Hammer- advanced whitening - Ingredients : Sorbitol, Sodium Bicarbonate, Aqua, Hydrated Silica, Glycerin, Tetrasodium Pyrophosphate, Aroma, Sodium Lauroyl Sarcosinate, Sodium Saccharin, Cellulose Gum, Sodium Lauryl Sulfate, Limonene, CI 77891.

Colgate- sensitive pro-relief - Ingredients : arginine-8%, Hydrated silica, calcium carbonate, glycerin, water, bicarbonate, flavour, cellulose gum, sodium saccharin, FD&C blue no. 1 (CI 42090).

Colgate -enamel protect - Ingredients : Aqua, Sorbitol, Glycerin, Hydrated Silica, Potassium Nitrate, PEG-12, Sodium Lauryl Sulfate, Aroma, Cellulose Gum, Tetrasodium Pyrophosphate, Sodium Fluoride, Sodium Saccharin, Xanthan Gum, Eugenol, Limonene, CI 77891.

Colgate-cavity protection - Ingredients : Dicalcium Phosphate Dihydrate, Aqua, Glycerin, Sorbitol, PEG-12, Sodium Lauryl Sulfate, Aroma, Cellulose Gum, Sodium Monofluorophosphate, Tetrasodium Pyrophosphate, Sodium Saccharin, Calcium Glycerophosphate, Sodium Fluoride, Limonene.

Colgate total-plus whitening - Active Substances : Sodium Fluoride 0.32% w/w (1450ppm F) Triclosan 0.3% w/w; Other Ingredients: Copolymer, Dental Type Silica, Glycerol, Sorbitol, Sodium Laurilsulfate, 89303/89854 Flavour, Carmellose Sodium, Titanium Dioxide, Saccharin Sodium, Carrageenan, Sodium Hydroxide, Mica, Water, CI Food Blue 2 (E133).

Colgate-Sensitive whitening - Ingredients : Sodium Fluoride (1450 ppmF-), Aqua, Glycerin, Sorbitol, Hydrated Silica, Potsium Nitrate, PEG-12, Tetrasodium Pyrophosphate, PVM/MA Copolymer, Sodium Lauryl Sulfate, Aroma, Cellulose Gum, Sodium Hydroxide, Sodium Saccharin, Xanthan Gum, Eugenol, Limonene, CI 77891, CI 42051.

Listerine Total Care Mouthwash : Aqua, Alcohol, Sorbitol, Aroma, Poloxamer 407, Benzoic Acid, Eucalyptol, Methyl Salicylate, Thymol, Menthol, Sodium Fluoride, Zinc Chloride, Sucralose, Sodium Saccharin, Sodium Benzoate, Benzyl Alcohol, CI 16035, CI 42090, Contains Sodium Fluoride (0.022% w/v 100 ppm F)

Aquafresh Mild and Minty Mouthwash : Aqua, Alcohol, Aroma, Cocamidopropyl Betaine, Sodium Saccharin, Cetylpyridinium Chloride, Sodium Fluoride, PEG-60 Hydrogenated Castor Oil, Sodium Bicarbonate, CI 42051, CI 75810, CI 15985

### DISCLOSURE OF INVENTION

This invention concerns a novel composition, combined with standard constituents of toothpastes and mouthwashes, for use in dental and oral hygiene, infections and inflammations.

The following are the active ingredients of the toothpaste and mouthwash :
Pelargonium sidoides extract
Vitamin A
Vitamin C
Vitamin E
Niacinamide
Folic acid
Zinc
Coenzyme Q10
Arginine
Calcium carbonate
Fluoride
Strontium chloride
Papain

The active ingredients are combined with standard constituents of toothpastes or mouthwashes. These additional ingredients can be altered and adapted to need, by either adding further ingredients, substituting ingredients, or by eliminating existing ingredients.

In order to maximize the effect of the composition, after brushing, additional paste containing the active composition may be applied directly to the teeth and gums for 1 to 2 minutes before rinsing.

The following is an example of the additional ingredients of the formulation when used in a toothpaste : sweetener (such as sorbitol or sodium saccharin), colouring (such as Aqua), flavouring (such as spearmint or menthol), aroma, water, mild abrasives (such as mica or sodium bicarbonate), sodium lauryl sulfate (foaming agent), cellulose gum (texturant).

The following is an example of the additional ingredients of the formulation when used in a mouthwash : alcohol (carrier for the flavour and as an anti-bacterial), sweetener (such as sorbitol or sodium saccharin), colouring (such as Aqua), flavouring (such as spearmint or limonene), aroma, water, sodium benzoate (preservative).

The following are the reasons for the inclusion of the active ingredients in the toothpaste and mouthwash:
Pelargonium sidoides extract - The genus Pelargonium comprises approximately 280 known species, of which 80% are grown in the interior of South Africa. Plant species are distinguished by the colour of the flowers, the shape of the leaves, and the pollen colour. Pelargonium sidoides is a small geranium-like plant that grows in a rosette from thick and very dark brown underground roots that grow up to 15cm in length. Sparsely branched stems grow from the base and display deep red to black flowers, and the species is distinguished by the cordate or heart shaped leaves. The pollen of the plant species is yellow. In contrast, Pelargonium reniforme has magenta red to black flowers, kidney shaped leaves, and white to green pollen. Both plant species may be found along the sea coast and interior portions of South Africa. Because of poor seed viability, a clonal method of propagation has been developed to reduce pressure on natural populations. The plant species indigenous to areas of South Africa are widely used by traditional healers of the Zulu, Basuto, Xhosa, and Mfengi tribes to treat dysentery, diarrhea, hepatic complaints, wounds, colds, fatigue, fevers, generalized weakness, and infections of the respiratory tract including tuberculosis. Western use of the Pelargonium sidoides and Pelargonium reniforme species to treat tuberculosis is traced back to the Englishman Major Charles Stevens in 1897 when he was treated by a tribal healer with an extract from the roots of Pelargonium species. Originally, the plant source of the drug was identified as Pelargonium reniform. However, further botanical investigations discovered the closely related species Pelargonium sidoides, which is now predominately used for therapeutic purposes. Chemical analyses have led to characterization of about 65 metabolites including phenolic and cinnamic acids, tannins, flavonoids, and coumarins in both plant species. Extracts of Pelargonium sidoides are composed of 6 main components that account for 70% to 80% of the total weight : substituted and unsubstituted oligomeric prodelphinidins, monomeric and oligomeric carbohydrates, minerals, peptides, purine derivatives, and highly substituted benzopyranones. Nearly 230 components have been detected in each of the plant species essential oils, with sesquiterpenes the most abundant. Coumarin, 5,6-dimethoxy, 7-hydroxy-coumarin, and several related ethers, glycosides, and sulfates have been isolated from both species of Pelargonium. Many of these coumarins also are found in the roots of other South African Pelargonium species. Pelargonium sidoides contains similar flavonoids and phenolics. The high tannin content (about 9%) may justify the plant's use in GI complaints in cases of bacterial toxin-induced secretory diarrhea. The broad spectrum of activity against viruses and bacteria may be caused by the coumarins and phenolic acids. Pelargonium sidoides extract may be useful in oral infective disorders as it has antiviral (herpes simplex virus), antibacterial (Staphylococcus aureus, Streptococcus pneumoniae), and antifungal (Candida albicans) activity against the causative organisms of oral disorders like gingivitis, periodontitis, glossitis and oral candidiasis.

Vitamin A - Vitamin A as retinyl palmitate and its fomation of retinol can lead to an enrichment of vitamin A in buccal mucosal cells. In this way metaplastic alterations of the buccal mucosal epithelium might be prevented or reversed by the application of topical retinyl palmitate. Vitamin A can reduce gingival bleeding activity and can be effective in the treatment of mild periodontitis.

Vitamin C - Vitamin C can reduce the total supragingival calculus score and reduce the number of bleeding sites.

Vitamin E - Topical vitamin E may be an effective therapy in patients with chemotherapy-induced oral mucositis.

Coenzyme Q10 : Topical application may improve adult periodontitis.

Folic acid - Folic acid appears to have an influence on gingival health via local influence. There is significant improvement in gingival inflammation. Topically applied folinic acid may be effective in facilitating resolution of oral lesions. Niacinamide - Niacinamide appears to have an influence on gingival health via local influence. There is significant improvement in gingival inflammation.

Zinc - Zinc citrate can enable significant inhibition of dental plaque formation, hence suggesting a new antiplaque and possible multi-benefit dentifrice for oral hygiene. Zinc in certain forms can reduce oral malodor.

Arginine - Arginine may help to provide significantly increased dentin hypersensitivity relief.

Calcium carbonate - Calcium carbonate may help to provide significantly increased dentin hypersensitivity relief.

Fluoride - Fluoride helps to prevent cavities, and has effects on the formation of dental enamel and bones.

Strontium chloride - Strontium Chloride may be an effective means for reducing the discomfort and pain engendered by thermal and tactile stimuli in patients with dentinal hypersensitivity.

Papain - Papain is a proteolytic enzyme with low specificity, suitable for cleaning the salivary protein plaque that has a whitening effect on the tooth surfaces.

### INDUSTRIAL APPLICABILITY

According to this invention, there is a composition, combined with standard constituents of toothpastes and mouthwashes, for use in dental and oral hygiene, infections and inflammations. In its optimal embodiment the composition consists, in the form of a toothpaste or mouthwash : Pelargonium sidoides extract, Vitamin A, Vitamin C, Vitamin E, Folic acid, Niacinamide, Zinc, Coenzyme Q10, Arginine, Calcium carbonate, Fluoride, Strontium chloride and Papain, combined with standard constituents of toothpastes or mouthwashes.

## Claims

1. A composition for dental and oral infections and inflammations that is in the form of a toothpaste or mouthwash, in which the active ingredients are Pelargonium, vitamin A, vitamin C, vitamin E, folic acid, niacinamide, zinc, coenzyme Q10, arginine, calcium carbonate, fluoride, strontium chloride, and papain.

2. A composition according to Claim 1 which is combined with the following in toothpastes : sweetener, colouring, flavouring, aroma, water, sodium bicarbonate, sodium lauryl sulfate, cellulose gum, and the following in mouthwashes : alcohol, sweetener, colouring, flavouring, aroma, water, sodium benzoate.

3. A composition according to Claims 1 or 2 that is combined with other constituents of toothpastes or mouthwashes.

4. A composition according to any one of claims 1 to 3 that is only in the form of a toothpaste.

5. A composition according to any one of claims 1 to 3 that is only in the form of a mouthwash.

## Patentansprüche

1. Zusammensetzung gegen Zahn- und Mundinfektionen und -entzündungen, die in Form einer Zahnpasta oder eines Mundwassers vorliegt, wobei die Wirkstoffe Pelargonium, Vitamin A, Vitamin C, Vitamin E, Folsäure, Niacinamid, Zink, Coenzym Q10, Arginin, Calciumcarbonat, Fluorid, Strontiumchlorid und Papain sind.

2. Zusammensetzung nach Anspruch 1, die mit den Folgenden in Zahnpasten kombiniert ist: Süßungsmittel, Farbmittel, Geschmacksmittel, Aromastoff, Wasser, Natriumbicarbonat, Natriumlaurylsulfat, Cellulosegummi und den Folgenden in Mundwässern: Alkohol, Süßungsmittel, Farbmittel, Geschmacksmittel, Aromastoff, Wasser, Natriumbenzoat.

3. Zusammensetzung nach den Ansprüchen 1 oder 2, die mit anderen Bestandteilen von Zahnpasten oder Mundwässern kombiniert ist.

4. Zusammensetzung nach einem der Ansprüche 1 bis 3, die nur in Form einer Zahnpasta vorliegt.

5. Zusammensetzung nach einem der Ansprüche 1 bis 3, die nur in Form eines Mundwassers vorliegt.

## Revendications

1. Composition pour infections et inflammations bucco-dentaires, se présentant sous la forme d'un dentifrice ou d'un bain de bouche, dans laquelle les constituants actifs sont le Pelargonium, la vitamine A, la vitamine C, la vitamine E, l'acide folique, la niacinamide, le zinc, le coenzyme Q10, l'arginine, le carbonate de calcium, le fluorure, le chlorure de strontium et la papaïne.

2. Composition selon la revendication 1, en combinaison avec les constituants suivants dans des dentifrices : édulcorants, colorants, arômes, eau, bicarbonate de soude, sodium lauryl sulfate, gomme de cellulose, et les constituants suivants dans les bains de bouche : alcool, édulcorants, colorants, arômes, eau, benzoate de sodium.

3. Composition selon les revendications 1 ou 2, en combinaison avec d'autres constituants de dentifrices ou de bains de bouche.

4. Composition selon l'une quelconque des revendications 1 à 3, se présentant uniquement sous la forme d'un dentifrice.

5. Composition selon l'une quelconque des revendications 1 à 3, se présentant uniquement sous la forme d'un bain de bouche.
